# EUROPÄISCHE PATENTSCHRIFT

(11) **EP 0 933 061 B1**
(45) Veröffentlichungstag und Bekanntmachung des Hinweises auf die Patenterteilung: **20.10.2004**
(21) Anmeldenummer: 99101818.5
(22) Anmeldetag: 28.01.1999
(51) Int. Cl.: A61B 5/03

(54) **Messung des intrakraniellen Druckes**
Measurement of the intracranial pressure
Mesure de la pression intracrânienne

(30) Priorität: 29.01.1998 DE 19803411; 08.04.1998 DE 19815664; 17.11.1998 DE 19852839
(43) Veröffentlichungstag der Anmeldung: 04.08.1999
(73) Patentinhaber: DWL Elektronische Systeme GmbH, 78354 Sipplingen (DE)
(72) Erfinder: Paulat, Klaus, Prof. Dr., 78354 Sipplingen (DE); Zolondz, Jürgen, 78315 Radolfzell (DE)
(74) Vertreter: Hiebsch, Gerhard F., Dipl.-Ing.

(56) Entgegenhaltungen:
- GB-A- 2 156 997
- US-A- 4 841 986
- US-A- 4 971 061

## Beschreibung

Die vorliegende Erfindung betrifft eine Erfindung zur intrakraniellen Druckmessung in einem Schädel eines Lebewesens.

Aus dem Stand der Technik sind verschiedene Vorgehensweisen bekannt, den Druck im Schädel eines Menschen (den intrakraniellen Druck ICP) zu messen, wobei herkömmlicherweise eine Miniaturdrucksonde (ICP-Sonde) nach Öffnung der Schädelkalotte eingesetzt wird und so die Registrierung des absoluten Druckwertes über einen längeren Zeitraum ermöglicht. Allerdings ist hierfür ein operativer Eingriff notwendig, der, neben dem hohen Aufwand, Infektionsgefahr in sich birgt und insbesondere bei Screening-Verfahren, also vorgeschalteten oder nicht rein diagnostischen bzw. therapeutischen Schritten, einen Menschen über Gebühr belastet.

Eine Vorrichtung zur intracraniellen Druckmessung ist ferner aus der US 4,841,986 bekannt.

Aufgabe der vorliegenden Erfindung ist es, eine Vorrichtung zur intrakraniellen Druckmessung in einem Schädel eines Lebewesens zu schaffen, die eine einfache Bedienung, Messwerterfassung und -auswertung gestattet und sich damit insbesondere für Screening-Anwendungen eignet. Insbesondere ist zudem eine solche Vorrichtung zu schaffen, die auf einfache Weise mit existierenden Anzeige- bzw. Patientendateneinheiten zusammenwirkt und so insbesondere derartige Ausgabevorrichtungen auch um eine neue Funktionalität, nämlich die Bereitstellung von nicht-invasiv gemessenen und aufbereiteten Daten zum Schädelinnendruck und zur Compliance, ergänzt.

Die Aufgabe wird durch die Vorrichtung mit den Merkmalen des Patentanspruches 1 gelöst.

Vorteilhaft ermöglicht es die erfindungsgemäße Vorrichtung, zuverlässig und schnell den in einer Proportionalbeziehung zum Schädelinnendruck stehenden Druck im Gehörgang sowie dessen zeitlichen Verlauf zu messen, und aus dem ausgewerteten Messsignal Rückschlüsse auf den intrakraniellen Druck sowie eine zeitliche Entwicklung dieses Druckes zu ziehen.

Die vorliegende Erfindung eignet sich durch ihre konstruktive und bedienungstechnische Einfachheit insbesondere auch zur Bedienung durch nicht medizinisch geschultes Personal und ist damit besonders günstig für langfristige, weitläufige und/oder routinemäßige Untersuchungen an einer großen Anzahl von Lebewesen, wobei dann eine weitere Auswertung der erfindungsgemäß erhaltenen Signale und Daten durch einen behandelnden Mediziner erfolgen kann.

Vorteilhafte Weiterbildungen der Erfindung sind in den Unteransprüchen beschrieben.

So ist es besonders bevorzugt, den erfindungsgemäßen Druckaufnehmer außerhalb des Gehörganges anzuordnen und diesen mit dem Gehörganginneren durch eine geeignete Druckleitung, etwa einen Schlauch, zu verbinden. Vorteilhaft könnte ein solcher Schlauch dann etwa dicht an die Verschlussmittel angekoppelt werden.

Generall liegt es im Rahmen der Erfindung, die Messwerterfassung im Bezug zu einer regelmäßigen Periode, insbesondere den Herzzyklus einer zu messenden Person, zu setzen. Erfindungsgemäß hat sich herausgestellt, dass eine weiter bevorzugt im Periodenabstand regelmäßige Auswertung vorbestimmter Messwerte innerhalb eines Zyklus die Bestimmung von Kenngrößen bzw. ausgewerteten Signalen erlaubt, die wiederum einen Rückschluss auf die intrakraniellen Druckverhältnisse zulassen.

Genauer gesagt liegt es im Umfang der Erfindung, die Auswerteeinheit so zu gestalten, dass diese charakteristische Absolutwerte und/oder einen charakteristischen Amplitudenhub zwischen einem minimalen und einem maximalen Druck-Messwert innerhalb einer Periode ermittelt; dieser Amplitudenhub steht in einem proportionalen, kausalen Zusammenhang mit dem intrakraniellen Absolutdruck.

Darüber hinaus liegt es im Rahmen weiterer, bevorzugter Weiterbildungen der Erfindung, weitere Extremwerte des Messwertverlaufs (innerhalb einer Messperiode) zu erfassen, und auch bezüglich dieser weiteren Extremwerte durch entsprechende Ausbildung der Auswerteeinheit das Erzeugen charakteristischer Verhältniswerte bzw. Amplitudenhübe zu ermöglichen. Ferner liegt es im Rahmen der Erfindung, zusätzliche Information dadurch bereitzustellen, dass die Auswerteeinheit weiterbildungsgemäß auch zum Erfassen eines zeitlichen Abstandes zwischen charakteristischen Extremwerten des Druckverlaufs innerhalb einer Messperiode ausgebildet ist und Auswertesignale ausgibt und/oder verrechnet, die diesem zeitlichen Abstand entsprechen.

Neben der erfindungsgemäß vorgesehenen Möglichkeit zur Analyse des Messwertverlaufes innerhalb einer, z.B. durch Herzschlag vorgegebenen, Periode liegt es im Rahmen der Erfindung, alternativ oder zusätzlich die gewünschte Information über den intrakraniellen Druck bzw. dessen Verlauf bereitzustellen, indem ein langfristiger Verlauf des Messwertes (lang gegenüber etwa einer Herzperiode) gemessen und ausgegeben wird.

Weiterhin liegt es im Rahmen der Erfindung, weiterbildungsgemäß Mittel vorzusehen, mit welchen etwa die Trommelfell-Vorspannung gemessen und zum beabsichtigten Ermitteln des Gehirndruckes ausgewertet werden kann: So ist es zum einen möglich, durch gezieltes Einbringen eines vorbestimmten Gas- oder Fluidvolumens in den abgeschlossenen oder den gegenüberliegenden Gehörgang (wodurch auch eine gezielte Volumen- bzw. Druckerhöhung im Gehirn bewirkt wird) die Reaktion des den Gehörgang endseitig verschließenden Trommelfells zu messen und so in die Lage versetzt zu sein, Rückschlüsse auf die (die Trommelfellspannung beeinflussende) Drucksituation im Gehirn zu ziehen und ggf. durch die zusätzliche Information über die Volumenänderung im Gehirn auf die Compliance zu schließen. Genauer gesagt ist die Auswerteeinheit so ausgebildet, dass diese charakteristische, druckbeeinflussende Reaktionen des Trommelfells erfassen, auflösen und so ausgewertet anbieten kann, dass auch auf diesem Wege Rückschlüsse eines Betrachters auf den intrakraniellen Druck möglich sind. Alternativ zu dieser Ausbildung zur Feststellung der Trommelfellspannung ist es möglich, die weiterbildungsgemäß zusätzlich vorgesehenen Kompensationsmittel so einzusetzen, dass durch gezielte Druckapplikation ein Ausgleich des herzschlagbedingten Pulsierens des Trommelfells erfolgt, so dass dann unmittelbar von der Trommelfelloberfläche, etwa durch einen Laser od.dgl., eine Information über die vorliegende Spannung gewonnen werden kann.

Die im ersten Fall, d.h. der gezielten zusätzlichen Druckaufbringung in den Gehörgang, einzusetzenden Vorrichtungselemente sind insbesondere Schallgeber oder der bereits mit den Verschlussmitteln in der Ohröffnung verbundene Druckschlauch, der durch gezielte, manuelle Kompression die Volumenänderung herbeiführt.

Auswerteseitig ist es besonders bevorzugt, das elektrisch auswertbare Signal in einen vom Herzschlag bereinigten Gleichspannungsanteil sowie einen Wechselspannungsanteil zu zerlegen und beide Kanäle parallel zur verbesserten Informationsgewinnung auszuwerten.

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in
- Fig. 1:: ein schematisches Blockschaltbild einer erfindungsgemäßen Vorrichtung zur intrakraniellen Druckmessung gemäß einer ersten, bevorzugten Ausführungsform;
- Fig. 2:: einen Signalverlauf charakteristischer, aufgenommener Drucksignale über eine Periode und
- Fig. 3:: ein Schaltbild eines Oszillators mit kapazitivem Spannungsteiler, der zur Erzeugung eines auswertbaren Nutzsignals aus einem kapazitiv wirkenden Bewegungsaufnehmer gemäß einer zweiten Realisierungsform der Erfindung ausgebildet ist.

Verschlussmittel in Form eines Ohrstopfens od.dgl. Element sind so ausgebildet, dass sie luftdicht abschließend in die Gehörgangöffnung eines menschlichen Ohrs eingesetzt werden können. Der Ohrstopfen 10 ist über einen Druckschlauch 12 mit einem extern vorgesehenen Druckaufnehmer 14 versehen, der über den Druckschlauch 12 in Verbindung mit dem vom Ohrstopfen 10 verschlossenen Gehörgang steht.

Der Druckaufnehmer 14 wandelt das gemessene Drucksignal in eine auswertbare, elektrische Spannung um, die zweikanalig zur weiteren Auswertung aufbereitet und dann einer (in der Figur nicht gezeigten) Auswert- und Anzeigeeinheit zugeführt wird.

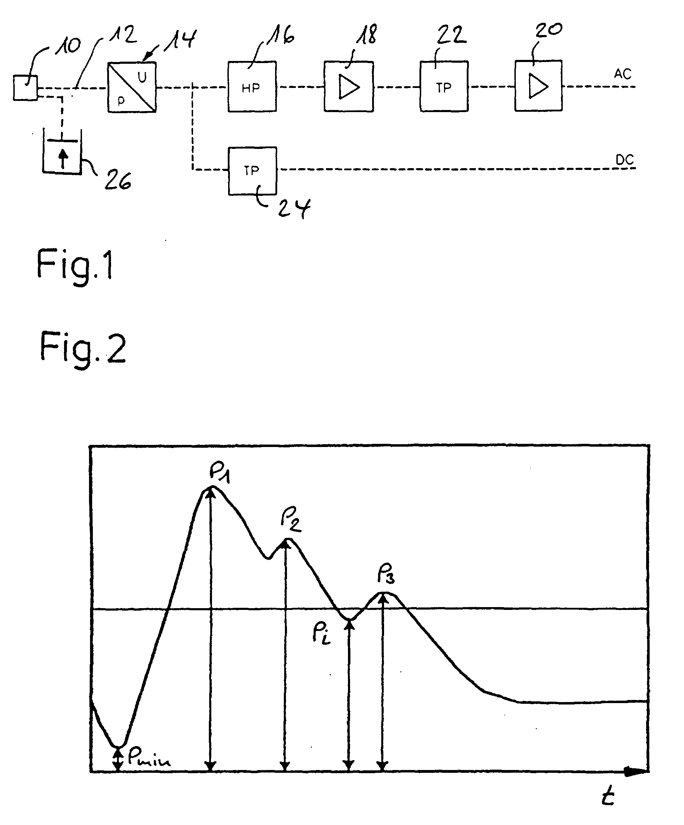

Genauer gesagt wird in einem ersten Zweig der Wechselspannungsanteil mittels eines Hochpasses 16, der eine Grenzfrequenz von beispielsweise 0,5 Hz aufweist, gefiltert, in nachfolgenden Verstärkerstufen 18, 20 um etwa den Faktor 20 bis 50 verstärkt und mittels eines Tiefpasses (TP) 22 mit einer oberen Grenzfrequenz von maximal 30 Hz in seiner Bahnenbreite auch nach oben hin beschränkt. Das aus diesem Zweig resultierende AC-Signal steht dann für eine weitergehende Signalaufbereitung, etwa mittels einer Sample-Schaltung (Takt z.B. 200 Hz, Auflösung 12 BIT) zur Verfügung, wobei hierdurch die Grundlage für weitergehenden, rechnerischen Signalauswertung des in Fig. 2 exemplarisch gezeigten Signals und zum Ausgeben entsprechender ICP-Druck-Information für eine Bedienperson einer (nicht gezeigten) Anzeigeeinheit geschaffen wird.

In einem zweiten Auswertezweig wird das Ausgangssignal des Druckaufnehmers 14 mittels eines Tiefpasses 24 einer Grenzfrequenz von beispielsweise 0,5 Hz gefiltert und steht dann unmittelbar als DC-Signal für eine weitere Auswertung zur Verfügung, die, gegenüber einer Herzperiode, auf einen längeren Zeitpunkt gerichtet ist.

Unter Bezug auf Fig. 2 wird nunmehr eine typische, durch den Druckaufnehmer 14 zu erfassende Signalform beschrieben, die in der gezeigten Weise mit nachgeschalteter Signalauswertung das Bestimmen des Gehirndruckes bzw. dessen Verlauf ermöglicht.

Die Fig. 2 verdeutlicht den exemplarischen Verlauf des elektronisch auswertbaren Drucksignals des Druckaufnehmers 14 über eine vom Herzschlag (Herzzyklus) bestimmte Periode eines Menschen.

Es zeigt sich, dass der bezogen auf eine Referenzlinie jeweils gemessene Druckwert P ein anfängliches Minimum Pₘᵢₙ aufweist und daraufhin steil zu einem Maximalwert P₁ ansteigt; im weiteren Kurvenverlauf folgen charakteristische, in ihrer Tendenz absteigende Schwankungen mit nachfolgenden Maxima P₂ und P₃. Ein vor P₃ liegender, minimaler Extremwert Pᵢ existiert häufig als tiefer Einschnitt (Inzisur) im Kurvenverlauf (lokales Minimum).

Durch die erfindungsgemäße Vorrichtung ist ein solcher charakteristischer Druckkurvenverlauf der signalanalytischen Auswertung zugänglich, wobei insbesondere die absoluten Pegel sowie der maximale Signalhub, nämlich das Verhältnis von P₁ zu Pₘᵢₙ bzw. der Abstand zwischen diesen charakteristisch sind, ebenso wie das Verhältnis von P₁ zu P₂. Insbesondere ist das erste dieser Amplitudenverhältnisse unmittelbar proportional zum intrakraniellen Druck, so dass aus dieser Messung eine unmittelbare Berechnung und Anzeige des Gehirndruckes bzw., bei mehrerern, aufeinanderfolgenden Perioden, dessen Verlaufs, möglich ist. Zusätzlichen Einfluss bzw. eine zusätzliche Möglichkeit zur Bestimmung des Absolutdruckes im Rahmen der Erfindung ergibt sich aus der Analyse eines zeitlichen Abstandes zwischen den aufeinanderfolgenden Maxima P₁, P₂ und P₃.

Gegenüber der Darstellung in Fig. 2, die einen typischen Wechselsignalverlauf im ersten Zweig gemäß Fig. 1 beschreibt, ist der Gleichspannungsverlauf in seinen möglichen Schwankungen wesentlich träger und wird üblicherweise über einen mehrminütigen bis gar mehrstündigen Zeitraum überwacht, um insbesondere auch Rückschlüsse auf Langfrist-Veränderungen des Gehirndruckes ziehen zu können.

Schließlich zeigt die Darstellung der Fig. 1 einen schematisch dargestellten Druck- bzw. Volumengeber 26, mit welchem bei eingesetztem Ohrstopfen 10 ein zusätzliches Volumen eines Gases oder eines Fluids in den geschlossenen Gehörgang eingebracht werden kann mit dem Ergebnis, dass auf das Trommelfell am dem Ohrstopfen 10 entgegengesetzten Ende ein zusätzlicher Druck aufgebracht wird, dessen Wirkung auf das Trommelfell, in Form von Anstiegzeit, Reaktionszeit od.dgl. dann wiederum über die in der Fig. 1 dargestellte Messwertkette erfasst und nachfolgend ausgewertet werden kann.

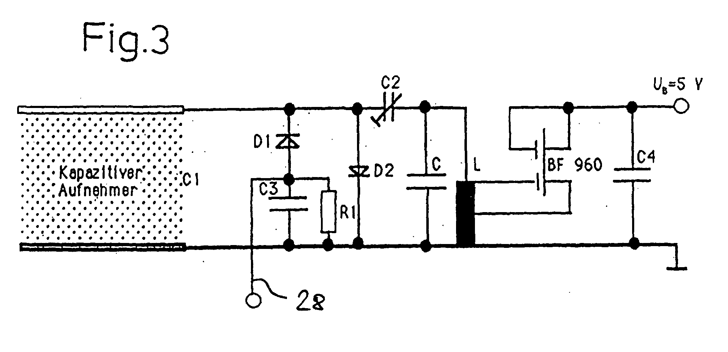

Fig. 3 zeigt zum zweiten, unabhängigen Erfindungskomplex eine typische Realisierungsform einer der auf den Schädel abnehmbar aufsetzbaren Vorrichtung zur Erfassung der Relativbewegung zugeordneten Signalaufbereitung. Genauer gesagt ist der in der Fig. 3 schematisch gezeigte kapazitive Aufnehmer C1 das Bewegungselement, welches so auf dem Schädel befestigt ist, das als Reaktion auf die Relativbewegung zweier Schädelplatten eine kapazitive Änderung von C1 auftritt (C1 besitzt im dargestellten Beispiel eine Kapazität von z.B. 20 pF). Mittels eines einen kapazitiven Spannungsteiler aufweisenden Oszillators wird am Signalausgang 28 ein zur weiteren Auswertung geeignetes und vorgesehenes Spannungssignal erzeugt, welches analog der in Fig. 1 dem Druckaufnehmer 14 nachgeschalteten Beschaltung weiter aufbereitet und dann einer rechnergestützten Signalverarbeitung zugeführt werden kann. Der etwa als Wechselspannungssignal erhaltene Signalverlauf entspricht dem in Fig. 2 dargestellten.

Typische Parameter für die Schaltung der Fig. 3 sind: Entladewiderstand R1 240 kOhm, Ladekondensator C3 22 nF; Schwingkreis-Kondensator C 150 pF; Trimmkondensator C2 10....30 pF; Siebkondensator C4 82 nF; Schwingkreis-Induktivität L 23µH; Gleichrichterdioden D1, D2 z.B. 1N 4148.

Die vorliegende Erfindung ist nicht auf die beschriebenen Ausführungsformen beschränkt. So ist es insbesondere möglich, die in den Figuren nicht gezeigte, der Signalaufbereitung nachgeschaltete Auswerteeinheit so auszubilden, dass diese, neben der absoluten und/oder relativen Schädeldruckmessung, eine Compliance-Ermittlung durchführt. Zu diesem Zweck wird das gezielt -- etwa mittels der Einheit 26 in Fig. 1 -- applizierte Volumen (welches insoweit auch auf das Gehirn bzw. den Schädelinnenraum wirkt) in Beziehung zu einer durch die Volumenänderung erreichten Änderung des Schädeldruckes gesetzt, und die Compliance ist damit ermittelbar. Durch dieses nicht-invasive Vorgehen bietet es sich darüber hinaus an, eine solche Compliance-Messung über einen großen Bereich der ICP und/oder einen längeren Zeitraum vorzunehmen, was mit bekannten, invasiven Vorgehensweisen zur Complicance-Messung nicht möglich ist, indem etwa mittels eines Kipptisches der ICP kontinuierlich erhöht und, bei periodischen Volumenapplikationen, kontinuierlich der gesamte Verlauf der Compliance ausgezeichnet wird.

Als besonders günstige Ausführungsform erweist sich in diesem Zusammenhang die Ausnutzung der arteriellen Windkesselfunktion, wobei das Problem der Erfassung des hierdurch periodisch auf den Schädelinnenbereich applizierten Volumens vorteilhaft und erfindungsgemäß mittels transkranieller Ultraschallsonographie gelöst wird. (Anlyse des sog. Power-Dopplersignals auf Querschnitts- bzw. Cross-Section-Index-Informationen eines relevanten, bevorzugt arteriellen Gefäßes.)

Gemäß einer weiteren, bevorzugten Ausbildung der Erfindung wird der Effekt ausgenutzt, dass die Trommelfellspannung proportional zum menschlichen Hirndruck ist. Damit kann, um etwa ein gut auswertbares Kriterium über den absolut-ICP bei Beginn einer kontinuierlichen Messung zu erhalten, der an sich bekannte Stapedes-Reflex, etwa durch ein Druck- oder Schallereignis auf dem kontralateralen Ohr, erzeugt wird, und die durch den Reflex erfolgende Lageänderung des Trommelfells meßseitig mit dem bekannten Druckaufnehmer erfasst wird.

## Patentansprüche

1. Vorrichtung zur intrakraniellen Druckmessung an einem Schädel eines Lebewesens, mit zum luftdichten Verschließen eines Gehörganges in die Ohröffnung einsetzbaren Verschlussmitteln (10),
einem den Verschlussmitteln (10) zugeordneten Druckaufnehmer (14), der zum Erfassen eines im verschlossenen Gehörgang vorliegenden Druckes und zum Erzeugen eines elektrisch auswertbaren Signals als Reaktion darauf ausgebildet ist,
und einer dem Druckaufnehmer (14) nachgeschalteten Auswerteeinheit, die zum Auswerten des Signals so ausgebildet ist, dass eine einem intrakraniellen Druck und dessen zeitlichem Verlauf entsprechende Information über eine Anzeigeeinheit ablesbar und/oder von einer Ausgabeeinheit so ausgebbar ist, dass Feststellungen über einen intrakraniellen Absolutdruck sowie dessen Entwicklung über einen vorbestimmten Zeitraum durch eine Bedienperson möglich sind,
**dadurch gekennzeichnet, dass**
Mittel zum Erfassen eines Herzzyklus vorgesehen sind und die Auswerteeinheit zum periodischen Auswerten mindestens eines innerhalb einer durch den Herzzyklus definierten Periode erhaltenen charakteristischen Messwerts (Pₘᵢₙ, P₁, P₂, Pᵢ, P₃) in Form eines Extremwerts im Druckkurvenverlauf und/oder eines Verhältnisses aufeinander folgender Extremwerte ausgebildet ist.

2. Vorrichtung nach Anspruch 1, **dadurch gekennzeichnet, dass** der Druckaufnehmer (14) den Verschlussmitteln im eingesetzten Zustand im Inneren des Gehörgangs zugeordnet ist.

3. Vorrichtung nach Anspruch 1, **dadurch gekennzeichnet, dass** der Druckaufnehmer (16) außerhalb des Gehörgangs vorgesehen ist und mit dem abgeschlossenen Gehörgang über eine Druckleitung (12) verbunden ist.

4. Vorrichtung nach einem der Ansprüche 1 bis 3, **dadurch gekennzeichnet, dass** die Auswerteeinheit zum Bestimmen eines Amplitudenhubs zwischen einem minimalen (Pₘᵢₙ) und einem maximalen (P₁) Messwert innerhalb einer vorbestimmten Messperiode ausgebildet ist.

5. Vorrichtung nach Anspruch 4, **dadurch gekennzeichnet, dass** die Auswerteeinheit zum Erfassen eines auf den maximalen Messwert (P₁) folgenden Extremwerts (P₂) im zeitlichen Verlauf des Drucks und zum Bestimmen eines weiteren Amplitudenhubs und/oder zeitlichen Abstands zwischen dem maximalen Messwert (P₁) und dem folgenden Extremwert (P₂) ausgebildet ist.

6. Vorrichtung nach einem der Ansprüche 1 bis 5, **dadurch gekennzeichnet, dass** die Auswerteeinheit zum Erfassen eines zeitlichen Abstandes zwischen aufeinanderfolgenden Extremwerten im zeitlichen Verlauf des Drucks innerhalb einer vorbestimmten Messperiode ausgebildet ist.

7. Vorrichtung nach einem der Ansprüche 1 bis 6, **dadurch gekennzeichnet, dass** die Auswerteeinheit zum Erfassen einer relativ zu einer insbesondere durch einen Herzzyklus bestimmten Periode langsamen und von dieser unbeeinflussten und/oder gemittelten Änderung des Druckes ausgebildet ist.

8. Vorrichtung nach einem der Ansprüche 1 bis 7, **gekennzeichnet durch** Mittel (26) zum Bestimmen und Applizieren eines Gas- und/oder Fluidvolumens in den verschlossenen Gehörgang, in den gegenüberliegenden Gehörgang oder in das Gehirn, wobei die Auswerteeinheit zum Erfassen einer Amplitude, einer Anstiegsgeschwindigkeit und/oder einer Reaktionszeit einer als Reaktion auf das Applizieren entstehenden Druckänderung im Gehörgang ausgebildet ist.

9. Vorrichtung nach Anspruch 8, **dadurch gekennzeichnet, dass** die Mittel zum Applizieren automatisch oder manuell betätigbare, volumenveränderliche Elemente sind.

10. Vorrichtung nach Anspruch 8, **dadurch gekennzeichnet, dass** die Mittel zum Applizieren eine Querschnittsund/oder Volumenmesseinheit aufweisen, die zum Erfassen einer durch die Windkesselfunktion des Arteriensystems bedingten periodischen Volumenänderung im Gehirn ausgebildet ist.

11. Vorrichtung nach einem der Ansprüche 1 bis 10, **gekennzeichnet durch** Mittel zum Erfassen einer gehirndruckbedingten Auslenkung des Trommelfells, die eine Laser- und/oder optische Reflexionseinheit aufweisen, zum Erzeugen charakteristischer Daten über einen Grad der Auslenkung des Trommelfells ausgebildet sind und mit Schwingungsmitteln zum Kompensieren einer einem Herzpuls entsprechenden, periodischen Auslenkung des Trommelfells **durch** ein periodisches, in den Gehörgang eingebrachtes Drucksignal zusammenwirken.

12. Vorrichtung nach einem der Ansprüche 1 bis 11, **dadurch gekennzeichnet, dass** die Auswerteeinheit eine Hochpass- und/oder Bandpasseinrichtung (16; 16, 22) zum Erzeugen eines Wechselspannungs-Nutzsignals aufweist, dessen Frequenz im Bereich zwischen 0,1 und 50 Hz liegt, und/oder eine Tiefpasseinrichtung (24) zum Erzeugen eines Gleichspannungs-Nutzsignals aufweist, deren Grenzbereich bei kleiner 0,5 Hz liegt.

## Claims

1. Device for measuring the intracranial pressure within the skull of a living being, comprising sealing means (10) which can be inserted into the ear opening for the airtight sealing of an auditory canal, a pressure transducer (14) associated with the sealing means (10) and designed to detect a pressure present in the sealed auditory canal and to generate as a reaction thereto a signal which can be evaluated electrically, and an evaluation unit arranged downstream of the pressure transducer (14) and designed to evaluate the signal in such a manner that information corresponding to an intracranial pressure and its variation with time can be read off a display unit and/or output by an output unit in such a manner that an operator can draw conclusions about an absolute intracranial pressure and its development over a predetermined period of time, **characterised in that** means are provided for detecting a cardiac cycle and the evaluation unit is designed to periodically evaluate at least one characteristic measured value (Pₘᵢₙ, P₁, P₂, Pᵢ, P₃) in the form of an extreme value in the pressure curve and/or a ratio of successive extreme values obtained within a period defined by the cardiac cycle.

2. Device according to claim 1, **characterised in that** the pressure transducer (14) is associated with the sealing means when they are inserted into the interior of the auditory canal.

3. Device according to claim 1, **characterised in that** the pressure transducer (14) is provided outside the auditory canal and is connected to the sealed auditory canal by means of a pressure line (12).

4. Device according to one of claims 1 to 3, **characterised in that** the evaluation unit is designed to determine an amplitude swing between a minimum (Pₘᵢₙ) and a maximum (P₁) measured value within a predetermined measuring period.

5. Device according to claim 4, **characterised in that** the evaluation unit is designed to detect an extreme value (P₂) following the maximum measured value (P₁) as the pressure varies with time and to determine a further amplitude swing and/or time interval between the maximum measured value (P₁) and the following extreme value (P₂).

6. Device according to one of claims 1 to 5, **characterised in that** the evaluation unit is designed to detect a time interval between successive extreme values as the pressure varies with time within a predetermined measuring period.

7. Device according to one of claims 1 to 6, **characterised in that** the evaluation unit is designed to detect a change in the pressure which is slow relative to a period determined, in particular, by a cardiac cycle, is not influenced thereby and/or is averaged.

8. Device according to one of claims 1 to 7, **characterised by** means (26) for determining and applying a gas and/or fluid volume to the sealed auditory canal, to the opposite auditory canal or to the brain, the evaluation unit being designed to detect an amplitude, an increase in speed and/or a reaction time of a change in the pressure in the auditory canal produced as a reaction to the application.

9. Device according to claim 8, **characterised in that** the application means are automatically or manually actuated elements of variable volume.

10. Device according to claim 8, **characterised in that** the application means comprise a unit for measuring the cross section and/or volume, designed to detect a periodic change in volume in the brain due to the windkessel function of the arterial system.

11. Device according to one of claims 1 to 10, **characterised by** means for detecting displacement of the eardrum due to cerebral pressure, comprising a laser and/or optical reflection unit, designed to generate characteristic data about the degree of displacement of the eardrum and cooperating with vibration means in order to compensate for periodic displacement of the eardrum corresponding to a heartbeat by means of a periodic pressure signal introduced into the auditory canal.

12. Device according to one of claims 1 to 11, **characterised in that** the evaluation unit comprises a high-pass and/or band-pass device (16; 16, 22) for generating a useful alternating voltage signal the frequency of which is in the range of between 0.1 and 50 Hz, and/or a low-pass device (24) for generating a useful direct voltage signal the limit range of which is less than 0.5 Hz.

## Revendications

1. Dispositif de mesure de la pression intracrânienne sur un crâne d'être vivant, comprenant
des moyens d'obturation (10) pouvant être introduits dans l'orifice auriculaire, afin d'assurer la fermeture hermétique d'un conduit auditif, un capteur de pression (14) associé à l'un des moyens d'obturation (10), lequel est configuré pour mesurer la pression présente dans un conduit auditif fermé et pour générer en réponse un signal électrique exploitable, et une unité d'exploitation connectée en aval du capteur de pression (14), laquelle, pour exploiter le signal, est configurée de telle sorte qu'une information correspondant à une pression intracrânienne et à son évolution dans le temps puisse apparaître sur un dispositif d'affichage et/ou être diffusée depuis une unité de sortie, et permette à un opérateur des constatations concernant une pression intracrânienne absolue et son évolution sur une période de temps prédéterminée ;
**caractérisé en ce que** des moyens d'enregistrement d'un cycle cardiaque sont prévus et **en ce que** l'unité d'exploitation est configurée de manière à permettre l'exploitation périodique d'au moins une valeur de mesure caractéristique (Pₘᵢₙ, P₁, P₂, Pᵢ, P₃) obtenue dans l'une des périodes définies par le cycle cardiaque sous forme d'une valeur extrême dans le schéma des courbes de pression et/ou sous forme d'un rapport entre des valeurs extrêmes consécutives.

2. Dispositif selon la revendication 1, **caractérisé en ce que** le capteur de pression (14) est associé aux moyens d'obturation à l'état monté à l'intérieur du conduit auditif.

3. Dispositif selon le revendication 1, **caractérisé en ce que** le capteur de pression (16) est prévu à l'extérieur du conduit auditif et est relié au conduit auditif obturé par une conduite de pression (12).

4. Dispositif selon l'une des revendications 1 à 3, **caractérisé en ce que** l'unité d'exploitation est configurée de manière à déterminer une élongation maximale d'amplitude comprise entre une valeur de mesure minimale (Pₘᵢₙ) et une valeur de mesure maximale (P₁) dans une période de mesure prédéterminée.

5. Dispositif selon la revendication 4, **caractérisé en ce que** l'unité d'exploitation est configurée pour enregistrer une valeur extrême (P₂) succédant à la valeur de mesure maximale (P₁) dans l'évolution temporelle de la pression et pour déterminer une autre élongation maximale d'amplitude et/ou un écart temporel entre la valeur de mesure maximale (P₁) et la valeur extrême (P₂) consécutive.

6. Dispositif selon l'une des revendications 1 à 5, **caractérisé en ce que** l'unité d'exploitation est configurée pour enregistrer l'écart temporel existant entre les valeurs extrêmes consécutives dans l'évolution temporelle de la pression à l'intérieur d'une période de mesure prédéterminée.

7. Dispositif selon l'une des revendications 1 à 6, **caractérisé en ce que** l'unité d'exploitation est configurée pour enregistrer une variation de la pression, lente, relativement à une période déterminée en particulier pendant un cycle cardiaque, et non influencée et/ou moyennée sur cette période.

8. Dispositif selon l'une des revendications 1 à 7, **caractérisé par** des moyens (26) de détermination et d'administration d'un volume de gaz et/ou de fluide dans le conduit auditif obturé, dans le conduit auditif opposé ou dans le cerveau, l'unité d'exploitation étant configurée pour enregistrer l'amplitude, la vitesse de montée et/ou le temps de réaction d'une variation de pression survenue sous forme de réaction à l'administration dans le conduit auditif.

9. Dispositif selon la revendication 8, **caractérisé en ce que** les moyens d'administration sont des éléments pouvant être actionnés automatiquement ou manuellement et à volume variable.

10. Dispositif selon la revendication 8, **caractérisé en ce que** les moyens d'administration présentent une unité de mesure de coupe transversale et/ou de volumes, qui est configurée pour enregistrer la variation périodique des volumes dans le cerveau, conditionnée par la fonction de réservoir d'air du système artériel.

11. Dispositif selon l'une des revendications 1 à 10, **caractérisé par** des moyens pour l'enregistrement de l'élongation du tympan en fonction de la pression cervicale, lesquels présentent une unité de réflexion laser et/ou optique, ces moyens étant configurés pour générer des données caractéristiques sur un degré d'élongation du tympan et coopérant avec des moyens de pulsation, pour compenser une élongation périodique du tympan correspondant à une pulsation cardiaque, par un signal de pression périodique émis dans le conduit auditif.

12. Dispositif selon l'une des revendications 1 à 11, **caractérisé en ce que** l'unité d'exploitation présente un dispositif passe-haut et/ou passe-bande (16 ; 16, 22), afin de générer un signal utile de tension alternative, dont la fréquence se situe dans la plage comprise entre 0,1 et 50 Hz, et/ou présente un dispositif passe-bas (24) pour générer un signal utile de tension continue, dont la plage limite est inférieure à 0,5 Hz.
